# EUROPEAN PATENT APPLICATION

(11) **EP 1 113 079 A2**
(43) Date of publication of application: **04.07.2001**
(21) Application number: 00128595.6
(22) Date of filing: 28.12.2000
(51) Int. Cl.: C12Q 1/68

(54) **A method for typing polymorphisms of bovine MHC class II genes**

(30) Priority: 28.12.1999 JP 37348399
(71) Applicant: Riken, Wako-shi, Saitama 351-0198 (JP); Aida, Yoko, Tsukuba-shi, Ibaraki 305-0035 (JP); Takeshima, Shinnosuke, Matsudo-shi, Chiba 271-0087 (JP)
(72) Inventor: Aida, Yoko, 3-105 Shareru tsukuba matsushiro, Tsukuba-shi, Ibaraki 305-0035 (JP); Takeshima, Shinnosuke, Matsudo-shi, Chiba 271-0087 (JP)
(74) Representative: Sternagel, Fleischer, Godemeyer & Partner Patentanwälte

(57) **Abstract**

A primer set used in PCR for accurately and rapidly typing polymorphisms of DNA encording BoLA-DRB3.2 which comprises:
(1) a reverse primer capable of amplifying all alleles of BoLA-DRB3.2; and
(2) a forward primer capable of amplifying all alleles in any one of two or more groups of alleles of BoLA-DRB3.2 wherein each of said groups comprises at least one allele, but incapable of amplifying allele(s) contained in the other group(s).

SEQUENCE LISTING
<110> The Institute of Physical and Chemical Research (RIKEN)
<120> A method for typing polymorphisms of bovine MHC Class II genes
<160> 14
<210> 1
<211> 38
<212> DNA
<213> Artificial Sequence
<220>
<223> Description of Artificial Sequence: Synthetic DNA
<400> 1
tgtaaaacga cggccagtag cacatttcct gcagtatc     38
<210> 2
<211> 42
<212> DNA
<213> Artificial Sequence
<220>
<223> Description of Artificial Sequence: Synthetic DNA
<400> 2
tgtaaaacga cggccagtag cacatttcct ggagtattct aa     42
<210> 3
<211> 39
<212> DNA
<213> Artificial Sequence
<220>

<223> Description of Artificial Sequence: Synthetic DNA

<400> 3

tgtaaaacga cggccagtag cacatttcct ggagtatta          39

<210> 4

<211> 39

<212> DNA

<213> Artificial Sequence

<220>

<223> Description of Artificial Sequence: Synthetic DNA

<400> 4

tgtaaaacga cggccagtag cacatttcct ggagtattg          39

<210> 5

<211> 36

<212> DNA

<213> Artificial Sequence

<220>

<223> Description of Artificial Sequence: Synthetic DNA

<400> 5

tgtaaaacga cggccagtca catttcctgg agtatg          36

<210> 6

<211> 38

<212> DNA

<213> Artificial Sequence

<220>

<223> Description of Artificial Sequence: Synthetic DNA

<400> 6

tgtaaaacga cggccagtgc acatttcctg gagtatc          37

<210> 7

<211> 38

<212> DNA

<213> Artificial Sequence

<220>

<223> Description of Artificial Sequence: Synthetic DNA

<400> 7

tgtaaaacga cggccagtag cacatttcct ggagtata 38

<210> 8

<211> 42

<212> DNA

<213> Artificial Sequence

<220>

<223> Description of Artificial Sequence: Synthetic DNA

<400> 8

tgtaaaacga cggccagtca catttcctgg agtattctac 40

<210> 9

<211> 39

<212> DNA

<213> Artificial Sequence

<220>

<223> Description of Artificial Sequence: Synthetic DNA

<400> 9

caggaaacag ctatgacccg ccgctgcaca gtgaaactc 39

<210> 10

<211> 28

<212> DNA

<213> Artificial Sequence

<220>

<223> Description of Artificial Sequence: Synthetic DNA

<400> 10

ggaattcctc tctctgcagc acatttcc 28

<210> 11

<211> 26

<212> DNA

<213> Artificial Sequence

<220>

<223> Description of Artificial Sequence: Synthetic DNA
<400> 11
tttaaattcg cgctcacctc gccgct 26
<210> 12
<211> 45
<212> DNA
<213> Artificial Sequence
<220>
<223> Description of Artificial Sequence: Synthetic DNA
<400> 12
tgtaaaacga cggccagtat tcctctctct gcagcacatt tcctg 45
<210> 13
<211> 18
<212> DNA
<213> Artificial Sequence
<220>
<223> Description of Artificial Sequence: Synthetic DNA
<400> 13
tgtaaaacga cggccagt 18
<210> 14
<211> 18
<212> DNA
<213> Artificial Sequence
<220>
<223> Description of Artificial Sequence: Synthetic DNA
<400> 14
caggaaacag ctatgacc 18

## Description

### Field of the Invention

The present invention relates to a method for rapidly typing bovine MHC Class II genes and a primer used for said method.

### Related Art

Major histocompatibility complex molecule (hereinafter abbreviated as "MHC") is a membrane protein which binds to an antigen peptide generated by intercellular decomposition of a protein antigen and presents the decomposed peptide to a T cell. Depending on their structural and functional differences, they are broadly classified into Class I and Class II. The most notable characteristic of MHC is that the molecule has uniquely high polymorphisms, and each polymorphism gathers in a groove accommodating an antigen peptide which exists at the edge of an extracellular domain of a MHC molecule. This polymorphisms defines an antigen peptide binding to MHC and causes individual differences regarding immune responses and sensitivity to diseases. Furthermore, many disease sensitive genes as well as the MHC gene group are mapped in the MHC region and some diseases are developed in correlation with the polymorphism of MHC. Accordingly, MHC has become focused as a marker of disease causing genes.

For the above reasons, the human MHC (HLA) gene group is one of the most investigated regions among the human genome, and in recent years, the outline of its genetic structure has been revealed through cloning using YAC and a cosmid vector. In addition, there are some reports regarding the role of their genetic products in immune responses, the existence of more than 500 alleles, and the association of the genes with the development of at least 100 different diseases typically include autoimmune diseases. With regard to the association with infectious diseases, it has been clarified that the number of HLA alleles or haplotypes significantly increases or decreases in smallpox, AIDS, Hansen's disease, malaria, adult T cell leukemia and the like.

A MHC Class II molecule is a membrane protein which exhibits significant polymorphisms and consists of α chain and β chain. The molecule is expressed only in specific cells including a B cell, microphage, monocyte, dendritic cell, skin Langerhans cell and the like. Both chains of Class II molecule, which consist of α1· β 1 domain-α2· β2 domain-membrane spanning domain-intercellular region, forms an immunoglobulin·like structure. Peptides, which are mainly derived from exogenous antigens such as bacteria and blood soluble antigens taken up by phagocytosis, bind to peptide-accommodating grooves of the extracellular domain of the Class II molecule and are presented to CD4 positive T cells. Among the CD4 positive T cells, helper T cells induce humoral immunity by B cells. Whilst, inflammatory T cells after being activated act to destroy substances in vacuoles rapidly by stimulating microphages.

It is presumed that the bovine MHC(BoLA) Class II genetic region is located on the chromosome 23 and consists of Class II, Class III, and Class I gene regions in the order from the centromere side. A major difference between BoLA Class II gene and the human or the mouse gene is that the gene is split into two sub·regions Class IIa and Class IIb by at least 15cM region lying between them. In the Class IIb sub-region, two genetic sub-regions DR and DQ are mapped. In the Class IIa sub·region, there have been identified DMA and DMB which stimulate the binding between MHC and antigens, LMP2 and LMP7 which are associated with the processing of antigens, a transporter gene TAP, and DNA, DOB, DIB, DYA and DYB whose protein expression and function are unknown. Thus, the second characteristic of BoLA Class II gene is that the gene corresponding to human DP gene sub-region has not been found, whilst the DY gene sub·region specific for ruminants exists whose correspondence with human genes remains unknown.

In the DR gene sub-region, each gene is located in the order of DRB1-DRB2-DRB3-DRA from the centromere side. A heterodimer consisting of α chain encoded by DRA and β chain encoded by DRB3 is expressed on a cell surface as a DR molecule, which induces strong antigen presenting ability. DRB3 is the most functional and polymorphous in BoLA genes and is revealed to have 96 alleles. In the DQ gene region, four different DQA genes as well as four different DQB genes have been identified so far. DQA genes or DQB genes are different to each other in the number or structure depending on a haplotype, and they form one or two DQ molecule(s) and induce weak antigen presenting ability.

### Disclosure of the Invention

As methods for typing BoLA-DRB3, some methods such as a PCR-RFLP, Analysis of a microsatellite adjuvant of DRB3 exon 2, Serological typing method and the like have been studied so far. However, none of these methods can be used to accurately determine alleles. Thus, an object of the present invention is to provide a method for accurately and rapidly determining a nucleotide sequence of bovine BoLA-DRB3 exon 2 (BoLA-DRB3.2), and typing its genetic polymorphism.

The inventors of the present invention conducted various studies to achieve the foregoing object, and as a result, they found that polymorphism of BoLA-DRB3.2 can be typed accurately and rapidly by grouping genes on the basis of the amino acid sequences of the first hypervariable region deriving from 96 different amino acid sequences of BoLA-DRB3.2; designing primers specific for each group; amplifying BoLA-DRB3.2 by the PCR (polymerase chain reaction) method; and then determining a nucleotide sequence of the PCR product. The present invention was achieved on the basis of these findings.

The present invention thus provides a primer set used in PCR for typing polymorphisms of DNAs encoding BoLA-DRB3.2 which comprises:
(1) a reverse primer capable of amplifying all alleles of BoLA-DRB3.2; and
(2) a forward primer capable of amplifying all alleles in any one of two or more groups of alleles of BoLA-DRB3.2 wherein each of said groups comprises at least one allele, but incapable of amplifying any allele(a) contained in the other group(s).

The forward primer preferably comprises a portion of a DNA sequence encoding the amino acid sequence of the first hypervariable region of BoLA-DRB3.2. Preferably, 96 different alleles of BoLA-DRB3.2 are classified into the two or more groups of the alleles of BoLA-DRB3.2. It is preferred that the 96 kinds of alleles of BoLA-DRB3.2 are classified into 8 groups, and that the forward primer is capable of amplifying all alleles in any one of the above 8 groups, but incapable of amplifying any alleles in the other groups. The forward primer preferably comprises a nucleotide sequence selected from the group consisting of the nucleotide sequences shown in SEQ ID NOS: 1, 2, 3, 4, 5, 6, 7 and 8. The reverse primer preferably comprises a nucleotide sequence shown in SEQ ID NO: 9.

In another aspect, the present invention provides a forward primer used in PCR for typing polymorphisms of DNA encoding BoLA-DRB3.2, which is capable of amplifying all alleles in any one of two or more groups of alleles of BoLA-DRB3.2 wherein each of said groups comprises at least one allels, but incapable of amplifying any allele(s) in the other group(s).

The primer preferably comprises a portion of a DNA sequence encoding the amino acid sequence of the first hypervariable region of BoLA-DRB3.2. Preferably, 96 different alleles of BoLA-DRB3.2 are classified into the two or more groups of the alleles of BoLA-DRB3.2. Preferably, the present invention provides a primer wherein 96 different alleles of BoLA-DRB3.2 are classified into 8 groups, and the primer is capable of amplifying all alleles in any one of the above 8 groups, but incapable of amplifying any alleles in the other groups. Preferably, the present invention provides a primer having a nucleotide sequence selected from the group consisting of the nucleotide sequences shown in SEQ ID NOS: 1, 2, 3, 4, 5, 6, 7 and 8.

Furthermore, the present invention provides a primer set of forward and reverse primers capable of amplifying all alleles of BoLA-DRB3.2, wherein the forward primer comprises a nucleotide sequence shown in SEQ ID NO: 12 and a reverse primer comprises a nucleotide sequence shown in SEQ ID NO: 9, and also a primer capable of amplifying all alleles of BoLA-DRB3.2 wherein the forward primer comprises a nucleotide sequence shown in SEQ ID NO: 12.

In further aspect, the present invention provides a method of typing polymorphisms of DNA encoding BoLA-DRB3.2 which comprises the steps of
(1) performing PCR by using the aforementioned primer set of the present invention, wherein a bovine genomic DNA or a DNA fragment thereof is used as a template; and
(2) where two PCR products are amplified in the step (1), directly sequencing each of the amplified products, and where one PCR product is amplified in the step (1), sequencing the amplified product by using a primer set capable of amplifying all alleles of BoLA-DRB3.2, and then comparing the resulting sequence(s) with known sequences of alleles. According to preferred embodiment of the aforementioned method, prior to the step (1), PCR is performed by using a primer set capable of amplifying all alleles of BoLA-DRB3.2 and using a bovine genomic DNA as a template, and then the step (1) is performed by using the resulting amplified product as a template.

### Brief Explanation of Drawing

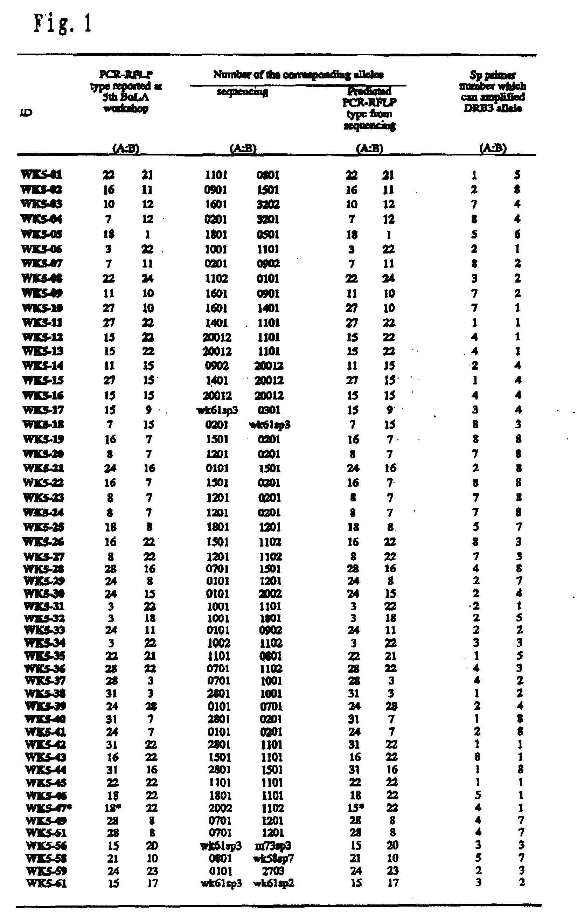

Figure 1 shows the result of comparison between nucleotide sequences of 53 different DNA samples typed by PCR-RFLP and nucleotide sequences determined by the method of the present invention.

### Best Mode for Carrying Out the Invention

The method of the present invention is characterized to compries the step of amplifying BoLA·DRB3.2 by means of PCR by using bovine genomic DNA as a template, wherein a primer is used as Forward (5') primer which is capable of amplifying all allelea contained in any one of the two or more groups of alleles (each of which comprises at least one allele) of BoLA·DRB3.2, but incapable of amplifying alleles contained in the other groups (herein may be referred to as "5' primer specific for a group"). By carrying out the step (2) after specific amplification of genes contained in a specific group, the polymorphisms can be very accurately and simply identified.

Samples used for preparing genomic DNA from a bovine individual include peripheral blood, organs and the like. Examples of the organs include tissue fragments such as lymph nodes. As a method of preparing genomic DNA from the above sample, any method which is available to those skilled in the art can be used. Where peripheral blood leucocyte or peripheral blood lymphocyte is used as a sample, for example, the method of Hughes *et al.* can be used (Hughes, S.H., *et al., Cell,* 15, pp. 1397-1410, 1978). Where organs are used, for example, an organ can be prepared by sodium dodecyl sulfate·phenol chloroform technique after frozen tissue fragments are sliced using scissors (Mcknight, G.S., *Cell,* 14, pp. 403-413, 1978). A simple extracting method of genomic DNA from cells may be also used.

BoLA-DRB3.2 is the most functional and polymorphous in the BoLA genes, and at least 96 different alleles have been identified so far, and more alleles may possibly be discovered in a future. Although the number of groups is not limited in the method of the present invention, preferable number of groups is 8 for the above 96 kinds of alleles. Typically, when PCR is performed using any one of 8 Forward (5') primers set forth below, all alleles contained in one of the 8 groups can be amplified, but alleles contained in the other groups cannot be amplified. The primers having such a feature can be designed on the basis of the amino acid sequence of the first hypervariable region of BoLA-DRB3.2.
sp1: 5'- TGT AAA ACG ACG GCC ACT AGC ACA TTT CCT GCA GTA TC -3'
sp2: 5'- TGT AAA ACG ACG GCC AGT AGC ACA TTT CCT GGA GTA TTC TAA -3'
sp3: 5'- TGT AAA ACG ACG GCC AGT AGC ACA TTT CCT GGA GTA TTA -3'
sp4: 5'- TGT AAA ACG ACG GCC AGT AGC ACA TTT CCT GGA GTA TTG -3'
sp5: 5'- TGT AAA ACG ACG GCC AGT CAC ATT TCC TGG AGT ATG -3'
sp6: 5'- TGT AAA ACG ACG GCC AGT GCA CAT TTC CTG GAG TAT C -3'
sp7: 5'- TGT AAA ACG ACG GCC AGT AGC ACA TTT CCT GGA GTA TA -3'
sp8: 5'- TGT AAA ACG ACG GCC AGT CAC ATT TCC TGG AGT ATT CTA C -3' (Nucleotide sequences of sp1 to sp8 are shown as SEQ ID NOS: 1 to 8, respectively.)

In step (1), a Reverse (3') primer capable of amplifying all alleles is used. An example of such primers includes the primer set forth below. Using the Reverse (3') primer and a Forward (5') primer specific for a group in combination, the group to which amplified genes belong can easily be identified.
DRB3B: 5'- CAG GAA ACA GCT AGT ACC CGC CGC TGC ACA GTG AAA CTC -3' (The nucleotide sequence of DRB3B is shown as SEQ ID NO: 9)

For carrying out the method of the present invention, it is desirable to perform the first PCR by means of a primer set capable of amplifying all alleles using genomic DNA as a template to equalize the template of each PCR solution. An example of the primer set includes the following set. It is possible to improve the accuracy of analysis by adopting this step.
ERB3 (SEQ ID NO: 10): 5'- GGA ATT CCT CTC TCT GCA GCA CAT TTC C -3'
HL031 (SEQ ID NO: 11) : 5'- TTT AAA TTC GCG CTC ACC TCG CCG CT -3'

After amplified products are obtained by using both Forward (5') primer specific for a group and Reverse (3') primer capable of amplifying all alleles, an analysis can be performed in accordance with the following procedures:
(A) An individual wherein two PCR products specific for a group are amplified:
   Each of the products are amplified alleles specific for a group, and the nucleotide sequences can easily be determined by the direct sequence method.
(B) An individual wherein only one PCR product specific for a group is amplified:
   The nucleotide sequences of amplified products, obtained by using a primer set capable of amplifying all alleles, are determined. Where two different kinds of alleles were amplified, accurate nucleotide sequences can easily be determined by comparing the results with nucleotide sequences of known alleles since candidates are limited. For verification, the nucleotide sequence of the amplified products may be determined by using a 5' primer specific for a group. The kinds of the primer sets capable of amplifying all alleles are not particularly limited. For example the following primer set may be used.

5' primer:
   DRB3ALL: 5'- TGT AAA ACG ACG GCC AGT ATT CCT CTC TCT GCA GCA CAT TTC CTG-3'
   (The nucleotide sequence of DRB3ALL is shown as SEQ ID NO: 12.)
3' primer:
   DRB3B: 5'- CAG GAA ACA GCT ATG ACC CGC CGC TGC ACA GTG AAA CTC-3'
   (The nucleotide sequence of DRB3B is shown as SEQ ID NO: 9.)

Where an individual having a BoLA-DRB3.2 allele wherein nucleotides 193 to 195 (3bp) are deleted, typing may sometimes be failed since the defective allele may interrupt the sequence corresponding to nucleotide 195 onward of an allele without the deletion. In such a case, both alleles can be determined by performing a direct sequencing from the antisense side to obtain the sequence of nucleotide 193 onward, and then combining the result with the nucleotide sequence corresponding to the nucleotides up to nucleotide 195 from the sense side,.

### Examples

The present invention will be more specifically explained with reference to the examples. However, the scope of the present invention is not limited to the following examples.

### Example 1

20 to 40 *µ*g of genomic DNA prepared from a bovine individual by simple extraction method was dissolved in 50 *µ*l×1 rTaq buffer (10mM Tris-HCl, 50mM KCl, 0.1% TritonX-100) containing 120 *µ*l of each dNTP, 1.5mM MgClz, 0.2 *µ*m of each primer and 2 units of recombinant Ta1 DNA elongation enzyme (rTaq)(TOYOBO). The solution was heated at 95°C for 5 minutes for denaturation, and then a cycle of reactions at 95°C for 50 seconds for denaturation, at 60°C for 50 seconds for annealing, and at 72°C for 50 seconds for elongation was repeated for 20 cycles. Then, elongation at 72°C for 2 minutes was performed. Primers used were those capable of specifically amplifying DRB3 gene exon 2 by PCR which encodes β 1 domain of bovine MHC Class II DR β chain (BoLA·DRβ).
ERB3: 5'- GGA ATT CCT CTC TCT GCA GCA CAT TTC C -3'
HL031: 5'- TTT AAA TTC GCG CTC ACC TCG CCG CT -3'

1 *µ*l of the above obtained PCR products was amplified by PCR (PCR-SSP), using primers each specific for respective 8 kinds of allele groups and a forward primer capable of amplifying all alleles.
(A) PCR specific for each allele group:
   25 *µ*l×1 GeneAmp^{r} Gold Buffer, containing 120 µl of each dNTP, 1.5mM MgCl₂, 0.2 *µ*M of a forward primer specific for an allele group, 200 *µ*l of a reverse primer, 1 unit of AmpliTaq Gold™ DNA elongation enzyme (PE Biosystems) and 1 µl of the above obtained PCR products, was heated at 95°C for 10 minute for denaturation, and then a cycle of reactions at 95°C for 1minute for denaturation, at 64°C for 30 seconds for annealing, and at 72°C for 30 seconds for elongation was repeated for 20 cycles. Then, elongation at 72°C for 5 minutes was performed.
      (a) Forward primers specific for respective allele groups:
         sp1: 5'- TGT AAA ACG ACG GCC AGT AGC ACA TTT CCT GCA GTA TC -3'
         sp2: 5' TGT AAA ACG ACG GCC AGT AGC ACA TTT CCT GGA GTA TTC TAA -3' sp3: 5'- TGT AAA ACG ACG GCC AGT AGC ACA TTT CCT GGA GTA TTA -3'
         sp4: 5'- TGT AAA ACG ACG GCC AGT AGC ACA TTT CCT GGA GTA TTG -3'
         sp5: 5'- TGT AAA ACG ACG GCC AGT CAC ATT TCC TGG AGT AGT -3'
         sp6: 5'- TGT AAA ACG ACG GCC AGT GCA CAT TTC CTG GAG TAT C -3'
         sp7: 5'- TGT AAA ACG ACG GCC AGT AGC ACA TTT CCT GGA GTA TA -3'
         sp8: 5'- TGT AAA ACG ACG GCC AGT CAC ATT TCC TGG AGT ATT CTA C -3'
      (b) Reverse primer:
         The following primer was designed as a primer capable of amplifying all alleles.
         DRB3B: 5'- CAG GAA ACA GCT ATG ACC CGC CGC TGC ACA GTG AAA CTC -3'
(B) PCR capable of amplifying all alleles:
   25 *µ*l × 1 GeneAmp^{R} Gold Buffer (PE Biosystema), containing 120 *µ*l of each dNTP, 1.5mM MgCl₂, forward primer capable of amplifying 0.2 *µ*M of all allele groups, 0.2*µ*l of reverse primer, 1 unit of AmpliTaq Gold™ DNA elongation enzyme and 1*µ*l of the above obtained PCR products, was heated at 95°C for 10 minutes for denaturation as a pretreatment, and then a cycle of reactions at 95°C for 1minute for denaturation, at 64°C for 30 seconds for annealing, and at 72°C for 30 seconds for elongation was repeated for 20 cycles. Then, elongation at 72°C for 5 minutes was performed.
   (a) Forward primer:
      DRB3ALL: 5'- TGT AAA ACG ACG GCC AGT ATT CCT CTC TCT GCA GCA CAT TTC CTG -3'
   (b) Reverse primer:
      DRB3B: 5'- CAG GAA ACA GCT ATG ACC CGC CGC TGC ACA GTG AAA CTC -3'

The PCR products were subjected to 2% agarose gel electrophoresis, then the agarose were stained with ethidium bromide. According to the number of detected bands, nucleotide sequences were determined as described below. The following primers were used as sequencing primers.
M13(-21) (SEQ ID NO:13): 5'- TGT AAA ACG ACG GCC AGT -3'
M13rev (SEQ ID NO:14): 5'- CAG GAA ACA GCT ATG ACC -3'
(A) When two amplified products are detected by PCR using the sequence specific primers
   1 µl each of the two PCR products, of which amplification were detected above, was subjected to a sequencing reaction using BigDye™ Terminator Cycle Sequencing Ready Reaction Kit (PE Biosystems) and half BD (GENPAK Ltd.). After 1 µl of PCR product, 1 *µ*l of 3 pmol primer, 1 *µ*l of Terminator Ready Reaction Mix, 3 *µ*l of half BD and 4 *µ*l of sterilized water were mixed, a cycle of reactions at 96°C for 10 seconds for denaturation, at 50°C for 5 seconds for annealing, and at 60°C for 4 minutes for elongation was repeated for 25 cycles according to the conditions described in an attached manual. Then, according to the manual, the amplified products were precipitated from ethanol, dissolved in 10 *µ*l of TSR for ABI310 sequencer, and then subjected to sequencing by using ABI310 (PE Biosystema). Each nucleotide sequence of the alleles was chacked by referring to the nucleotide sequences registered in a database to determine the sequence of alleles.
(B) When only one amplified product is detected by PCR using the sequence·specific primers
   (a) 1 *µ*l of amplified PCR product, obtained by PCR using primers capable of amplifying all alleles, was subjected to a sequencing reaction using BigDye™ Terminator Cycle Sequencing Ready Reaction Kit and half BD. On the basis of sequences in a state of a mixture of two kinds of alleles, each nucleotide sequence was determined by referring to a database.
   (b) When the latter half of a sequence is unreadable as a result of the sequencing

Some BoLA-DRB3 exon 2 alleles exist whose nucleotides 194 to 196 are deleted. When both of the defective allele and an alleles without the deletion are subjected to sequencing at the same time, it is difficult to determine a nucleotide sequence at nucleotide 194 onward when the forward primer is used, or a nucleotide sequence up to nucleotide 196 when the reverse primer is used. In such a case, a part of a sequence obtained by sequencing using the forward primer and a part of a sequence obtained by sequencing using the reverse primer are combined to determine nucleotide sequences in a state of a mixture of two kinds of alleles, and then each nucleotide sequence was determined by referring to a database.

The results are shown in Figure 1. The nucleotide sequences of 53 kinds of DNA samples, which were typed by PCR·RFLP reported in the 5^{th} International BoLA Workshop, completely matched to PCR·RFLP types.

As explained above, according to the method of the present invention, nucleotide sequences of bovine BoLA-DRB3 exon 2 (BoLA-DRB3.2) can be rapidly and accurately determined, and genetic polymorphisms can be efficiently typed.

## Claims

1. A primer set used in PCR for typing polymorphisms of DNAs encoding BoLA·DRB3.2 which comprises:
(1) a reverse primer capable of amplifying all alleles of BoLA-DRB3.2; and
(2) a forward primer capable of amplifying all alleles in any one of two or more groups of alleles of BoLA-DRB3.2 wherein each of said group comprises at least one allele, but incapable of amplifying any allele(s) in the other group(a).

2. The primer set according to claim 1 wherein a forward primer comprises a portion of a DNA sequence encoding an amino acid sequence of the first hypervariable region of BoLA·DRB3.2.

3. The primer set according to claim 1 or claim 2 wherein 96 kinds of alleles of BoLA-DRB3.2 are classified into the two or more groups of alleles of BoLA-DRB3.2.

4. The primer set according to any one of claims 1 to 3 wherein 96 kinds of alleles of BoLA-DRB3.2 are classified into 8 groups, and wherein the forward primer is capable of amplifying all alleles in any one of said 8 groups but incapable of amplifying any alleles in the other groups.

5. The primer set according to any one of claims 1 to 4 wherein the forward primer comprises a nucleotide sequence selected from the group consisting of the nucleotide sequences described in SEQ ID NOS: 1, 2, 3, 4, 5, 6, 7 and 8.

6. The primer set according to any one of claims 1 to 5 wherein the reverse primer comprises a nucleotide sequence described in SEQ ID NO: 9.

7. A forward primer used in PCR for typing polymorphisms of DNAs encoding BoLA·DRB3.2 which is capable of amplifying all alleles in any one of two or more groups of alleles of BoLA-DRB3.2 wherein each of said groups comprises at least one allele, but incapable of amplifying allele(s) in the other group(s).

8. The primer according to claim 7 which comprises a portion of a DNA sequence encoding an amino acid sequence of the first hypervariable region of BoLA-DRB3.2.

9. The primer according to claim 7 or 8 wherein 96 kinds of alleles of BoLA-DRB3.2 are classified into the two or more groups of alleles of BoLA-DRB3.2.

10. The primer according to any one of claims 7 to 9, wherein 96 kinds of alleles of BoLA·DRB3.2 are classified into 8 groups, and wherein said primer is capable of amplifying all alleles in any one of said 8 groups, but incapable of amplifying any alleles contained in the other groups.

11. The primer according to any one of claims 7 to 10 which comprises a nucleotide sequence selected from the groups consisting of the nucleotide sequences described in SEQ ID NOS: 1, 2, 3, 4, 5, 6, 7 and 8.

12. A primer set comprising a forward primer and a reverse primer capable of amplifying all alleles of BoLA·DRB3.2, wherein said forward primer comprises a nucleotide sequence described in SEQ ID NO: 12 and said reverse primer comprises a nucleotide sequence described in SEQ ID NO: 9.

13. A forward primer capable of amplifying all alleles of BoLA-DRB3.2, wherein said forward primer comprises a nucleotide sequence described in SEQ ID NO: 12.

14. A method for typing polymorphisms of DNA encoding BoLA-DRB3.2, which comprises the steps of:
(1) performing PCR by using the primer set according to any one of claims 1 to 6 and using a bovine genomic DNA or a DNA fragment thereof as a template; and
(2) where two PCR products are amplified in said step (1), directly sequencing each of the amplified products, and where one PCR product is amplified in said step (1), sequencing the amplified product by using a primer set capable of amplifying all alleles of BoLA-DRB3.2, and then comparing resulting sequence(s) with known sequences of alleles.

15. The method according to claim 14, wherein PCR is performed by using a primer set capable of amplifying all alleles of BoLA-DRB3.2 using a bovine genomic DNA as a template, and then said step (1) is performed by using a resulting amplified product as a template.
